# EUROPÄISCHE PATENTANMELDUNG

(11) **EP 0 940 180 A2**
(43) Veröffentlichungstag der Anmeldung: **08.09.1999**
(21) Anmeldenummer: 99102888.7
(22) Anmeldetag: 04.03.1999
(51) Int. Cl.: B01L 3/00, G01N 33/74

(54) **Vorrichtung zur Bestimmung von Testosteron**

(30) Priorität: 06.03.1998 DE 19809702
(71) Anmelder: HESCH, Rolf Dieter, Prof. Dr., D-78464 Konstanz (DE)
(72) Erfinder: HESCH, Rolf Dieter, Prof. Dr., D-78464 Konstanz (DE)
(74) Vertreter: Forstmeyer, Dietmar, Dr. rer. nat., Dipl.-Chem.

(57) **Zusammenfassung**

Eine Vorrichtung zur Bestimmung des Testosteronspiegels von Mensch und Tier umfaßt einen ersten Abschnitt zum Einbringen einer gesammelten Körperflüssigkeit, einen zweiten Abschnitt zum Durchführen einer Reaktion der Körperflüssigkeit mit einem Reaktionsträger, und einen dritten Abschnitt zum Anzeigen des Reaktionsergebnisses, wobei die Körperflüssigkeit mittels einer Vorrichtung vom ersten Abschnitt in den zweiten Abschnitt transportiert wird. Als Körperflüssigkeit wird vorzugsweise Speichel verwendet, der in einer Salivette gesammelt und in den ersten Abschnitt eingebracht wird, von wo er mittels einer Kompressionsvorrichtung über eine Verbindung vom ersten in den zweiten Abschnitt transportiert wird, so daß eine Reaktion des Speichels mit dem Reaktionsträger in Gang kommen kann.

## Beschreibung

Die Erfindung betrifft eine Vorrichtung zur Bestimmung von Testosteron und insbesondere eine Vorrichtung zur Messung des Testosteronspiegels von Mensch und Tier.

Gegenwärtig spielen sowohl in der Öffentlichkeit als auch in der klinischen Wissenschaft die Symptome der hormonabhängigen Gesundheit des Mannes eine zunehmende Rolle. Die hormonabhängige Gesundheit des Mannes wurde gegenüber der der Frau bisher vernachlässigt. Ein großes Problem stellt die Tatsache dar, daß es keinen "Männerarzt (Andrologen)" in der Weise wie einen "Frauenarzt (Gynäkologen)" gibt, die Probleme der hormonabhängigen Gesundheit sind weder beim Urologen noch beim Dermatologen und schon gar nicht beim Hausarzt angesiedelt. Es besteht daher ungenügender Kenntnisstand über die gestörte Gesundheit und die Gesundheitsschaden beim Mann, die durch absinkende Androgen-(Testosteron)-Produktion aus dem Hoden entstehen.

Neben dem altersmäßig langsamen Absinken der Testosteronproduktion kommt es im Leben des Mannes, seltener zwischen 20 - 45 Jahren, häufiger ab 45 - 50 Jahre episodisch über mehr oder weniger lange Zeitabstände (Wochen bis Monate) zu einem Absinken der Testosteronproduktion des Hodens mit entsprechenden biographischen Symptomen und organischen Krankheitszeichen. Diese Störung wird vorzugsweise durch psychische und körperliche Überlastung mit ungenügenden Ruhephasen hervorgerufen, ferner durch ungesunde Lebensweise und Belastung mit Toxinen (Zigaretten, Alkohol und andere). Während beim älter werdenden Mann das langsame Absinken der Testosteronproduktion aus dem Hoden durch ein langsames Nachlassen der Hodenfunktion an sich bedingt ist, so wird der episodisch auftretende Hormonmangel durch eine Depression des Zwischenhirns und der Hirnanhangdrüse hervorgerufen, so daß der Hoden nicht mehr ausreichend stimuliert wird. Dieses Krankheitsbild wird als "komplexer Androgenmangel" bezeichnet.

Ein Hauptproblem bei der Diagnostik des primären (hodenbedingten) Androgenmangels des Mannes und des "komplexen Androgenmangels" des Mannes besteht darin, daß das männliche Hormon Testosteron im Blut einer Tagesrhythmik unterliegt, wobei die Werte morgens zwischen 6:00 - 8:00 Uhr am höchsten und abends zwischen 18:00 - 21:00 Uhr am niedrigsten liegen. Dazwischen kommt es zum langsamen Abfall der Hormonkonzentration: die individuell außerordentlich variabel sein kann. Dies bringt es mit sich, daß es kaum "Normalwerte" der Konzentration männlichen Hormones gibt und übliche Blutabnahmen im Verlaufe des Tages ein ungenügend exaktes Bild geben, da keine geeigneten Referenzbereiche vorliegen.

Zusammenfassend kann daher festgestellt werden, daß es in Deutschland, aber auch in Europa, kaum andrologisch ausgebildete Ärzte gibt, so daß die hormonabhängige Gesundheit des Mannes vernachlässigt wird. Darüber hinaus bestehen große technische Probleme bei der Messung des männlichen Hormonspiegels.

Testverfahren zum Bestimmen des Testosteronspiegels eines Probanden sind bekannt, wobei bei den Nachweisverfahren der Testosteronnachweis durch Bindungsproteine (Ligandenbindungsassays), Antikörper (Enzymimmunoassays) oder Rezeptorproteine (Biosensor) geführt wird. So bietet die Firma DPC Biermann GmbH, Bad Nauheim als Beispiel eines Antikörpernachweises ein Testkit zur Verwendung in einer Arztpraxis an, mittels dem anhand des in Körperflüssigkeiten vorhandenen Testosterons der Testosteronspiegel des Probanden bestimmt wird. Als Körperflüssigkeiten kommen Speichel, Plasma, Serum oder Urin in Betracht. Bei dem Testverfahren konkurrieren Testosteron der Probe mit ¹²⁵I-markiertem Testosteron um eine begrenzte Anzahl von Bindungsstellen hochspezifischer Antikörper, die auf die Innenwandung von Polypropylenröhrchen immobilisiert sind. Nach Beendigung oder Gleichgewichtseinstellung der Reaktion wird das freie, nicht an die Antikörper gebundene Testosteron abgeschüttet oder abgesaugt und die Radioaktivität der leeren Röhrchen in einem Gammazähler gemessen.

Ferner ist es eine weitere Tatsache, daß die Frau Vorsorge-(Prävention)-maßnahmen eher wahrnimmt als der Mann, dieser beansprucht vorzugsweise die "Reparaturmedizin". Um den Mann zu einer Untersuchung seiner hormonabhängigen Gesundheit zu motivieren, bedarf es des Nachweises, daß zahlreiche, bei Männern oft beobachtete Symptome tatsächlich mit niedrigen Spiegeln von männlichem Hormon einhergehen. Um die "Ärztescheue" des Mannes zu umgehen, empfiehlt es sich daher, dem männlichen Charakter entsprechend, ein Meßinstrument anzubieten, welches durch Eigenmessung dem Mann erlaubt, sich Information über seinen hormonabhängigen Gesundheitszustand zu verschaffen. Hierzu ist es erforderlich, daß die Messung einmal morgens um etwa 8:00 Uhr und einmal abends um ca. 18:00 Uhr durchgeführt wird. Dies ist nur möglich, wenn die Messung als "Selbstmessung" durchgeführt werden kann.

Der Erfindung liegt daher die Aufgabe zugrunde, eine Vorrichtung zur Bestimmung des Testosteronspiegels von Mensch und Tier zu schaffen, mittels der auf einfache und bequeme Weise eine Bestimmung des Testosteronspiegels unabhängig von einer Arztpraxis oder dergleichen möglich ist.

Die Aufgabe wird durch den Gegenstand des Anspruchs 1 gelöst. Bevorzugte Ausgestaltungen der Erfindung sind Gegenstand der Unteransprüche.

Die erfindungsgemäße Meßvorrichtung zur Bestimmung des Testosteronspiegels von Mensch oder Tier umfaßt einen ersten Abschnitt zum Einbringen gesammelter Körperflüssigkeit, einen zweiten Abschnitt zum Durchführen einer Reaktion der Körperflüssigkeit mit einem Reaktionsträger, und einen dritten Abschnitt zum Anzeigen des Reaktionsergebnisses, wobei die Körperflüssigkeit mittels einer Vorrichtung vom ersten Abschnitt in den zweiten Abschnitt transportiert wird.

Bevorzugt wird in den ersten Abschnitt der Vorrichtung eine mit Körperflüssigkeit beladene Salivette eingebracht. Die Vorrichtung zum Transportieren der Körperflüssigkeit vom ersten in den zweiten Abschnitt kann durch eine Komprimiervorrichtung gebildet werden, die die Salivette komprimiert, so daß die Körperflüssigkeit über eine Verbindung von dem ersten Abschnitt in den zweiten Abschnitt transportiert wird. Vorzugsweise wird die Komprimiervorrichtung durch einen Kolbenmechanismus gebildet. Es kann jedoch auch beispielsweise ein Unterdruckmechanismus nach dem Prinzip einer Monovette oder jeder andere Mechanismus, der die Körperflüssigkeit in die Reaktionskammer bringt, verwendet werden.

Als Reaktionsträger können Testosteronnachweise mittels Bindungsproteine, Antikörper oder Rezeptorproteine verwendet werden.

Falls der Sammler der Körperflüssigkeit und die Reaktionsträger Einmalartikel sind, ist die Hygiene der Vorrichtung gesichert.

Vorzugsweise ist der Reaktionsträger als Schwellenwertgeber ausgelegt, der anzeigt, ob die gemessene Testosteronkonzentration oberhalb einer vorbestimmten Größe ist oder nicht.

Vorzugsweise wird Speichel als Körperflüssigkeit verwendet, allerdings können auch andere Körperflüssigkeiten wie Blut etc. verwendet werden.

Die Meßvorrichtung weist vorzugsweise eine zylindrische Form auf und kann relativ kompakt gehalten werden.

Vorteilhafterweise ermöglichst die Vorrichtung gemäß der Erfindung die Bestimmung von Testosteron im Speichel unabhängig von einer Praxis im Selbsttest, so daß der Mann oder die Person Information über seine hormonabhängige Gesundheit erhalten kann. Das Meßinstrument gibt eine "ja/nein"-Antwort darüber, ob die Konzentration von Testosteron über einem Minimalbereich liegt oder darunter und ferner, ob der tageszeitliche Rhythmus gestört ist. Je nach erhaltenem Meßwert kann der Mann dann einen Arzt zu einer Androgensubstitution (Hormonersatzbehandlung) aufsuchen.

Die Erfindung ist nachfolgend anhand der Zeichnung erläutert.

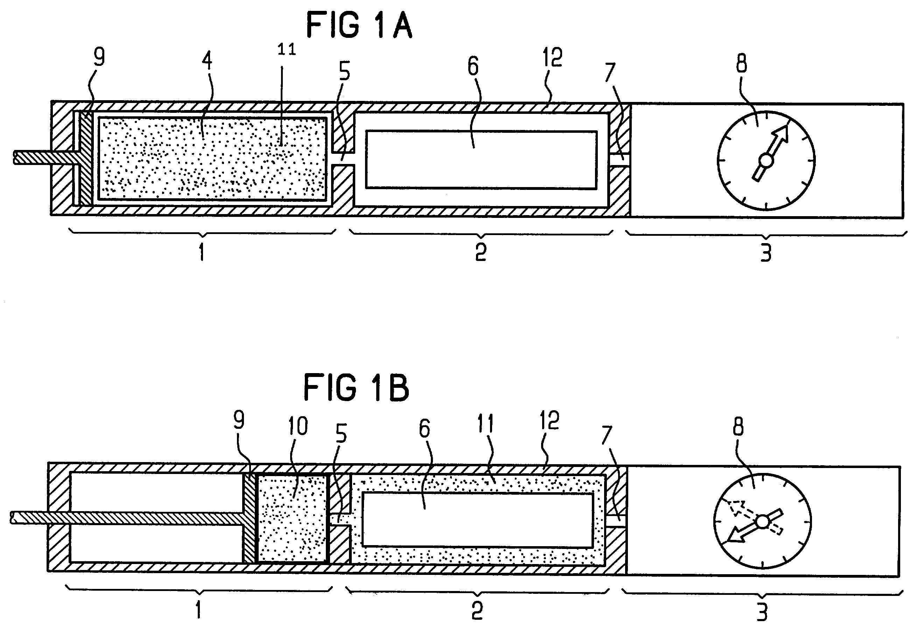

Fig. 1A zeigt eine schematische Darstellung der erfindungsgemäßen Meßvorrichtung in einem ersten Zustand mit der Körperflüssigkeit im ersten Abschnitt, und
Fig. 1B zeigt die Meßvorrichtung nach Fig. 1A in einem zweiten Zustand nach erfolgtem Transport der Körperflüssigkeit in den zweiten Abschnitt.

Figur 1A zeigt eine bevorzugte Ausführungsform einer Meßvorrichtung zur Selbstbestimmung des Testosteronspiegels, die im wesentlichen aus drei Abschnitten besteht, wobei der erste Abschnitt 1 als Speichelsammelabschnitt, der zweite Abschnitt 2 als Reaktionsabschnitt und der dritte Abschnitt als Anzeigeabschnitt dient. Dabei zeigt Figur 1A die Meßvorrichtung in einem ersten Zustand, in dem sich die zu analysierende Körperflüssigkeit 11 (punktiert dargestellt) im ersten Abschnitt befindet. Im ersten Abschnitt 1, der über eine Verbindung mit dem zweiten Abschnitt 2 verbunden ist, befindet sich eine mit Speichel getränkte Salivette 4. In dem zweiten Abschnitt 2 ist ein Reaktionsträger 6 angeordnet. Meßsignale werden Ober eine schematisch dargestellte Verbindung 7 zwischen dem zweiten und dem dritten Abschnitt auf eine Anzeigevorrichtung 8 gegeben. Die Meßvorrichtung weist ferner eine Komprimiervorrichtung 9 auf, mittels der die Körperflüssigkeit 11 von dem ersten in den zweiten Abschnitt 1, 2 transportiert werden kann. Die drei Abschnitte 1, 2, 3 sind in einem gemeinsamen Gehäuse 12, beispielsweise in der Form eines Zylinders, untergebracht.

Fig. 1B zeigt die Meßvorrichtung in einem zweiten Zustand. Mittels der Komprimiervorrichtung 9, beispielsweise in der Form eines Kompressionskolbens, wird die Salivette 4 zu einer komprimierten Salivette 10 verformt, so daß der darin enthaltene Speichel bzw. Körperflüssigkeit 11 durch die Verbindung 5 in den zweiten Abschnitt gedrückt wurde. Die Körperflüssigkeit 11 ist im zweiten Abschnitt punktiert dargestellt. Diese Körperflüssigkeit 11 reagiert mit dem Reaktionsträger nach einem der erwähnten Verfahren. Das Ergebnis der Reaktion wird durch die Anzeigevorrichtung 8 dargestellt, die über die Verbindung 7 mit dem Reaktionsträger 6 in Verbindung steht. Die Verbindung, d.h. die Datenübertragung vom Reaktionsträger 6 zu der Anzeigeeinheit 8 kann beispielsweise durch eine Signalverbindung, wie ein Draht (nicht dargestellt), in bekannter Weise erfolgen.

Im folgenden wird die Wirkungsweise der Meßvorrichtung unter Bezug auf beide Zeichnungen näher erläutert:

Zur Untersuchung der Körperflüssigkeit wird das Prinzip der "Salivette" genutzt. Die Salivette 4 besteht aus einem speziell gepackten, rundlichen Filtermaterial, welches zwischen Zähne und Wangenschleimhaut so verbracht wird, daß es sich mit Speichel vollsaugt. Eine solchermaßen vollgesaugte Salivette 4 kann dann durch Kompression oder Zentrifugation vom Speichel getrennt werden, dieser steht als lösliche Flüssigkeit zur Messung bestimmter Substanzen zur Verfügung. Eine mit Speichel getränkte Salivette 4 wird in den Speichelabschnitt 1 der beschriebenen Meßvorrichtung eingebracht. Mit dem Verschluß des Speichelsammelabschnitts 1 wird gleichzeitig ein Komprimiermechanismus wie ein Kolbenmechanismus 9 in der Weise in Gang gesetzt, durch den die Salivette 4 komprimiert wird. Dadurch wird der Speichel durch eine Verbindung 5, die den Speichelsammelabschnitt 1 mit dem Reaktionsabschnitt 2 verbinden, in den letzteren hinübergepreßt. Es muß nicht notwendigerweise der dargestellt Kolbenmechanismus verwendet werden. Dieser dient nur als Beispiel. Es kann beispielsweise auch ein Unterdruckmechanismus eingesetzt werden, durch den der Speichel vom Abschnitt 1 in den Reaktionsabschnitt 2 verbracht wird. Ferner ist auch ein Überdruckmechanismus, beispielsweise mittels einer Gaspatrone, verwendbar, der den Speichel vom Abschnitt 1 in den Reaktionsabschnitt 2 überführt.

Die Reaktionseinheit 2 umfaßt ein Reaktionssystem oder einen Reaktionsträger 6, welches Testosteron im Speichel messen kann. Der Speichel bzw. die Körperflüssigkeit 11 kann dabei auch in den dargestellten Reaktionsträger 6 eindringen (nicht dargestellt). Für den Reaktionsträger 6 bedient man sich von an sich bekannten Nachweismethoden für freies Testosteron beispielsweise durch Bindungsproteine (Ligandenbindungsassay), durch Antikörper (z.B. Enzymimmunoassay) oder Rezeptorproteine (z.B. Biosensor).

Die Nachweisreaktion von Testosteron im Speichel in der Reaktionseinheit 6 arbeitet nach dem Prinzip des "Cut off". Mit anderen Worten, die Nachweisreaktion zeigt an, ob die Konzentration für Testosteron oberhalb oder unterhalb eines kritischen Bereiches liegt, z.B. 3 ng/ml Gesamttestosteron oder entsprechend der Konzentration an freiem Testosteron in pg/ml. Die so erzielte Information wird über eine Datenverbindung (nicht dargestellt) auf eine Anzeigeeinheit 8 gegeben.

Speichelsammelabschnitt 1 und Reaktionsabschnitt 2 werden nach Ablauf der Reaktion mit Leitungswasser gereinigt, d.h. Speichelsammler (Salivette 4) und Reaktionsträger 6 sind Einmalartikel und werden nach abgelaufener Reaktion entsorgt. Die Reaktionsanzeige kann durch Farbumschlag erfolgen, z.B. wie bei klassischem Enzymimmunoassay oder durch Veränderung elektrischer Ströme wie bei Biosensoren, respektive durch eine andere geeignete Reaktionsanzeige, die beide Bereiche trennend darstellen kann.

Die Anzeige erfolgt in einer Anzeigeeinheit 8. Diese kann eine Mikrocomputer beinhalten, der Werte mit entsprechenden Daten von Messungen über mehrere Zeiträume aufsammeln kann.

Im Prinzip kann ein solches Instrument auch für Blut benutzt werden, analog den Blutzuckermeßgeräten wie sie heute schon zur Verfügung stehen. Dabei muß der Proband sich aber selbst stechen, um Blut zu gewinnen, dies stellt in aller Regel eine relativ große Hindernisschwelle dar.

Die Größe des Gerätes kann etwa der eines dicken Kugelschreibers bis hin zu einem Handy-Telefon entsprechen. Vorteilhafterweise kann die Meßvorrichtung daher auf Reisen mitgenommen werden.

### Bezugszeichenliste

- 1: erster Abschnitt
- 2: zweiter Abschnitt
- 3: dritter Abschnitt
- 4: Salivette
- 5: Verbindung
- 6: Reaktionsträger
- 7: Verbindung
- 8: Anzeigeeinheit
- 9: Komprimiervorrichtung
- 10: komprimierte Salivette
- 11: Körperflüssigkeit
- 12: Gehäuse

## Patentansprüche

1. Vorrichtung zur Selbstbestimmung des Testosteronspiegels von Personen oder Tieren,
**dadurch gekennzeichnet, daß** die Vorrichtung aufweist:
einen ersten Abschnitt (1) zum Einbringen gesammelter Körperflüssigkeit (11),
einen zweiten Abschnitt (2) zum Durchführen einer Reaktion der Körperflüssigkeit (11) mit einem Reaktionsträger (6), und
einen dritten Abschnitt (3) zum Anzeigen des Reaktionsergebnisses,
wobei die Körperflüssigkeit (11) mittels einer Vorrichtung (9) vom ersten Abschnitt (1) in den zweiten Abschnitt (2) transportiert wird.

2. Vorrichtung nach Anspruch 1, **dadurch gekennzeichnet,** daß in dem ersten Abschnitt (1) der Vorrichtung eine mit Körperflüssigkeit (11) beladene Salivette (4) eingebracht wird.

3. Vorrichtung nach Anspruch 2, **dadurch gekennzeichnet,** daß die Vorrichtung (9) zum Transportieren der Körperflüssigkeit vom ersten (1) in den zweiten Abschnitt (2) durch eine Komprimiervorrichtung (9) gebildet wird, die die Salivette (4) komprimiert, so daß die Körperflüssigkeit (11) über eine Verbindung (5) von dem ersten Abschnitt (1) in den zweiten Abschnitt (2) transportiert wird.

4. Vorrichtung nach Anspruch 3, **dadurch gekennzeichnet,** daß die Komprimiervorrichtung (9) durch einen Kolbenmechanismus gebildet wird.

5. Vorrichtung nach einem der vorangegangenen Ansprüche, **dadurch gekennzeichnet,** daß als Reaktionsträger (6) Testosteronnachweise mittels Bindungsproteine, Antikörper oder Rezeptorproteine verwendet werden.

6. Vorrichtung nach einem der vorangegangenen Ansprüche, **dadurch gekennzeichnet,** daß der Sammler (4) der Körperflüssigkeit und die Reaktionsträger (6) Einmalartikel sind.

7. Vorrichtung nach einem der vorangegangenen Ansprüche, **dadurch gekennzeichnet,** daß der Reaktionsträger (6) als Schwellenwertgeber ausgelegt ist, der anzeigt, ob eine Testosteronkonzentration oberhalb einer vorbestimmten Größe liegt oder nicht.

8. Vorrichtung nach einem der vorangegangenen Ansprüche, **dadurch gekennzeichnet,** daß Speichel als Körperflüssigkeit (11) verwendet wird.

9. Vorrichtung nach einem der vorangegangenen Ansprüche, **dadurch gekennzeichnet,** daß die Meßvorrichtung eine zylindrische Form aufweist.
